# EUROPEAN PATENT APPLICATION

(11) **EP 3 581 123 A1**
(43) Date of publication of application: **18.12.2019**
(21) Application number: 19180257.8
(22) Date of filing: 14.06.2019
(51) Int. Cl.: A61B 17/221, A61B 18/26

(54) **PERCUTANEOUS EXTRACTOR WITH LASER ASSEMBLY**

(30) Priority: 14.06.2018 US 201862684894 P
(71) Applicant: Cook Medical Technologies, LLC, Bloomington, IN 47404 (US)
(72) Inventor: CARDINALI, Sydney, Louisville, KY 40299 (US); GERSTNER, Megan, Gallatin, TN 37066 (US); SINK, Ashley, St. Louis, MO 63021 (US); STUBBLEFIELD III, Walter, Ashland, KY 41102 (US); WARDLE, Timothy, Youngstown, OH 44512 (US)
(74) Representative: Leach, Sean Adam

(57) **Abstract**

An extractor includes a rigid outer cannula forming a lumen extending between a proximal end and an opposing distal end of the outer cannula. An inner cannula is slidably positioned within the lumen of the outer cannula. The inner cannula forms a central passage along a longitudinal axis of the inner cannula extending between a proximal end and an opposing distal end of the inner cannula. A plurality of secondary passages are positioned about the central passage and extend along at least a portion of a length of the inner cannula between the proximal end and the distal end of the inner cannula. An annularly expanding and retracting extraction mechanism at the distal end of the inner cannula is operable to capture and extract kidney stones from within a lumen of a patient.

## Description

### CROSS-REFERENCE TO RELATED APPLICATIONS

This application claims priority from U.S. Provisional Application No. 62/684,894, filed on June 14, 2018, the entirety of which is hereby incorporated by reference herein.

### BACKGROUND

The example embodiments described herein relate generally to surgical retrieval devices and, more particularly, to surgical retrieval devices for capturing and retrieving or extracting stones, calculi, concretions, foreign bodies and the like from a human or veterinary patient. The surgical retrieval device may also be useful for biopsies and other surgical retrievals.

Minimally invasive surgical procedures have been developed for the removal of stones, calculi, concretions and the like from the biliary, vascular, and urinary systems, as well as for the removal or retrieval of foreign bodies from a variety of locations in the body. Such procedures avoid the performance of open surgical procedures such as, for example, an anatrophic nephrolithotomy. Minimally invasive procedures can instead employ percutaneous access, in which stones, calculi, concretions, foreign bodies and the like are removed through a percutaneously inserted access sheath. Several access routes are suitable, depending upon the specific system and the particular location in the system at which the stones, calculi, concretions, foreign bodies or the like are found. One access route that is infrequently used is direct percutaneous insertion of a retrieval device to remove calculi and kidney stones.

Without regard to the particular access route, percutaneous extraction may be based upon the use of catheters or similar devices to engage and remove the stones, calculi, concretions, foreign bodies and the like. Such catheters and devices typically comprise a hollow, flexible sheath and a plurality of wires positioned in and extendable from the sheath. The wires are joined or arranged so as to form a means, such as a basket or forceps for engaging the object to be retrieved when the wires are extended from the sheath. The wires may also form a continuum with the sheath. The engagement means, e.g., a basket, can be collapsed by withdrawing the wires into the sheath. A helical basket permits entry of the stone, for example, from the side of the basket, while an open ended ("eggwhip") basket allows a head-on approach to the stone, for example. Other retrievers and graspers can include forceps or can include a wire loop or snare for encircling the body to be removed. Such devices may be used in conjunction with a nephroscope to aid the physician in seeing the operating field. Using such a device also tends to limit the size of the cannula and basket used.

During some procedures, relatively large foreign bodies are found at the site that have to be fragmented into small body pieces before removal. To fragment the larger foreign body, a ureterscope is introduced into the patient through a body passageway, such as the ureter. The ureterscope includes an integral optical system, a working channel, and a controller to maneuver the ureterscope to the target site. The surgeon then uses a laser assembly including a laser fiber, such as a holmium laser fiber, to fragment the stone. The laser assembly is then withdrawn from within the working channel and a suitable grasper is introduced through the working channel to the target site to remove any stone fragments.

### SUMMARY

In one example embodiment, an extractor includes an outer cannula having a lumen extending between a proximal end and an opposite distal end of the outer cannula. An inner cannula is movably positioned within the outer cannula. The inner cannula has a proximal end configured to be retained exteriorly of a patient and a distal end configured to be inserted into the patient. The inner cannula includes a central passage extending along a longitudinal axis of the inner cannula between the proximal end and the distal end. A plurality of secondary passages are positioned about the central passage. Each secondary passage of the plurality of secondary passages extends between the proximal end and the distal end of the inner cannula. A handle has a first section coupled to the proximal end of the inner cannula and a second section coupled to the proximal end of the outer cannula. The handle is movable between a first position and a second position to move the inner cannula within the outer cannula. An extraction mechanism, such as an annularly expanding extraction mechanism, is operatively coupled to the distal end of the inner cannula. In certain example embodiments, the annularly expanding extraction mechanism includes a plurality of fixed flexure elements positioned about a periphery of the central passage in an annular array at a confining fixed position and having a flexure position spaced longitudinally outwardly therefrom. Each movable flexure element of a plurality of corresponding movable flexure elements has an end fixed with respect to the flexure position of a corresponding fixed flexure element of the plurality of fixed flexure elements and extending therefrom in a longitudinally movable and generally transversely confined relation to a receiving portion of an adjacent fixed flexure element of the plurality of fixed flexure elements, wherein each movable flexure element extends through the adjacent fixed flexure element and a respective secondary passage of the plurality of secondary passages to couple to the first section of the handle. The extractor may further comprise a laser assembly movably positioned within the central passage, the laser assembly having a laser fiber configured to extend distally with respect to the distal end of the inner cannula as the handle is moved to the second position. The handle may further comprise a first aperture in the first section and a ring positioned about the first aperture, and each movable flexure element is coupled to the ring. The extractor may further comprise a laser assembly movably positioned within the central passage, wherein the ring forms a passage coaxially aligned with the central passage, the passage configured to receive a proximal end portion of the laser assembly. The handle may be operatively coupled to the extraction mechanism such that a manual movement of the first section towards the second section in a first direction will effect a movement of the plurality of movable flexure elements in an outward direction with respect to the corresponding fixed flexure element to extend in an arcuately flexed condition beyond the flexure positions of the plurality of fixed flexure elements to cause the plurality of fixed flexure elements to flex transversely outwardly and create an expanded condition defined by transversely outwardly flexed fixed flexure elements interconnected by an annular series of arcuately movable flexure elements. The handle may be configured so that a manual movement of the handle in an opposite second direction will effect a movement of the movable flexure elements when in the expanded condition in a direction inwardly with respect to the corresponding fixed flexure elements to cause the expanded condition to progressively retract during which the transversely outwardly flexed fixed flexure elements are progressively less flexed transversely outwardly and the arcuately flexed movable flexure elements have a progressively less arcuate extent. The extractor may be configured so that, as the first section is urged toward the second section, the inner cannula translates through the lumen of the outer cannula in a distal direction to extend the extraction mechanism distally from the inner cannula. The handle may further comprise an intermediate section between the first section and the second section, the intermediate section including a biasing member operatively coupled to the first section and the second section to allow movement of the first section with respect to the second section. The first section may have an upper portion with a first aperture extending through a width of the upper portion and a ring positioned within the upper portion about the first aperture, wherein the ring is configured to receive each movable wire section that extends through a respective secondary passage. The extractor may further comprise a laser assembly, wherein the ring is configured to receive a proximal end of the laser assembly to retain the laser assembly coupled to the first section, the proximal end of the laser assembly having a connector configured to extend into the first aperture and securely couple to the first section. The second section of the handle may include an upper portion having a second aperture coaxially aligned with first aperture of the upper portion of the first section, wherein the laser assembly is coupled to the upper portion of the first section and extends through the second aperture in the upper portion of the second section to enter the inner cannula and as the first section and the second section move towards each other, the first aperture and the second aperture remain coaxially aligned to facilitate movement of the laser assembly through the inner cannula in a distal direction.

In one example embodiment, an extractor includes a rigid outer cannula forming a lumen extending between a proximal end and an opposing distal end of the outer cannula. An inner cannula is slidably positioned within the lumen of the outer cannula. The inner cannula forms a central passage along a longitudinal axis of the inner cannula extending between a proximal end and an opposing distal end of the inner cannula. A plurality of secondary passages are positioned about the central passage and extend along at least a portion of a length of the inner cannula between the proximal end and the distal end of the inner cannula. An annularly expanding and retracting extraction mechanism at the distal end of the inner cannula is operable to capture and extract kidney stones from within a lumen of a patient. The retracting of this extraction mechanism is optional. The extraction mechanism may comprises a plurality of coextensive flexible tubular members disposed in an annular array at the distal end of the inner cannula, each flexible tubular member of the plurality of coextensive flexible tubular members is positioned within an associated secondary passage of the plurality of secondary passages, each flexible tubular member extends distally from the corresponding secondary passage and each wire of a plurality of wires is associated with a respective flexible tubular member of the plurality of coextensive flexible tubular members, each wire defines a fixed wire section and a movable wire section, each fixed wire section extends within a respective flexible tubular member and each movable wire section extends distally from the respective flexible tubular member and into an adjacent flexible tubular member. Each movable wire section may extend through the adjacent flexible tubular member and passes through a respective secondary passage to operatively couple to the handle. The extractor may further comprise a laser assembly extending through the central passage, the laser assembly includes a flexible laser fiber configured to deliver focused holmium energy at the distal end of the inner cannula. The extractor may comprise a handle configured to operate the extraction mechanism, the handle includes a first section coupled to the inner cannula and a second section coupled to the outer cannula, wherein as the first section is urged toward the second section, the inner cannula translates through the outer cannula in a distal direction to extend the extraction mechanism distally from the inner cannula. The handle may further comprise an intermediate section between the first section and the second section, the intermediate section includes a biasing member operatively coupled to the first section and the second section to allow movement of the first section with respect to the second section. The first section may have an upper portion with a first aperture extending through a width of the upper portion and a ring positioned within the upper portion about the first aperture, wherein the ring is configured to receive each movable wire section that extends through a respective secondary passage. The ring may be configured to receive a proximal end of a laser assembly to retain the laser assembly coupled to the first section, the proximal end of the laser assembly having a connector configured to extend into the first aperture and securely couple to the first section. The second section of the handle may include an upper portion having a second aperture coaxially aligned with first aperture of the upper portion of the first second, wherein the laser assembly is coupled to the upper portion of the first section and extends through the second aperture in the upper portion of the second section to enter the inner cannula and as the first section and the second section move towards each other, the first aperture and the second aperture remain coaxially aligned to facilitate movement of the laser assembly through the inner cannula in a distal direction. A method for using an extractor to remove a kidney stone percutaneously may include providing a percutaneous tract to a target site in a kidney in a patient's body, the target site including the kidney stone. An outer cannula of the extractor is advanced through the percutaneous tract to the target site. An inner cannula of the extractor is advanced through the outer cannula until a distal end of the inner cannula reaches the targeted site. A handle of the extractor grasping by an operator to expand an extraction mechanism at the distal end of the inner cannula to engage and capture the kidney stone to be extracted. With the kidney stone positioned within the extraction mechanism, the kidney stone is fragmented with a laser assembly to facilitate removing smaller pieces of the kidney stone from within the patient's body. The inner cannula is withdrawn from the patient outwardly of the installed percutaneous tract with the kidney stone fragmented. Fragmenting the kidney stone with a laser assembly may further comprise operating a laser assembly with a distal end of the laser assembly extending distally from a central passage of the inner cannula to fragment the kidney stone. These and other methods of the present disclosure may be practiced ex vivo, for example on surgical training dummies, cadavers or donated organs, and so may not comprise methods of treatment of the human or animal body by surgery or therapy.

### BRIEF DESCRIPTION OF THE DRAWINGS

The detailed description is set forth with reference to the accompanying figures. The use of the same reference numbers in different figures indicates similar or identical items or features.
FIG. 1A is a plan view of an example percutaneous extractor, according to various embodiments;
FIG. 1B is a sectional view of a portion of the example percutaneous extractor shown in FIG. 1A, according to various embodiments;
FIG. 2 is a plan view of the example percutaneous extractor shown in FIG. 1 with an extractor device advanced from a distal end of an outer cannula of the example percutaneous extractor, according to various embodiments;
FIG. 3 is a plan view of the example percutaneous extractor shown in FIG. 1 with the extractor device in an open position, according to various embodiments;
FIG. 4 is a plan view of a distal portion of the example percutaneous extractor shown in FIG. 3, according to various embodiments;
FIG. 5 is a side view of the distal portion of the example percutaneous extractor shown in FIG. 4, according to various embodiments;
FIG. 6 is a perspective view of a distal portion of an inner cannula of the example percutaneous extractor shown in FIG. 1, according to various embodiments;
FIG. 7 is a side view of a distal portion of an alternative example percutaneous extractor, according to various embodiments;
FIG. 8 is a perspective view of a handle suitable for use with the example percutaneous extractor, according to various embodiments;
FIG. 9 is a plan view of a ring assembly of the handle shown in FIG. 8, according to various embodiments;
FIG. 10 is a sectional view of a portion of a laser assembly suitable for use with the example percutaneous extractor, according to various embodiments; and
FIG. 11 is a perspective view of an alternative example handle suitable for use with the example percutaneous extractor, according to various embodiments.

### DETAILED DESCRIPTION

Example embodiments are disclosed herein. It is understood, however, that the disclosed embodiments are merely exemplary and may be embodied in various and alternative forms. The figures are not necessarily to scale; some figures may be configured to show the details of a particular component. Therefore, specific structural and functional details disclosed herein are not to be interpreted as limiting but merely as a representative basis for the claims and/or teaching one skilled in the art to practice the embodiments.

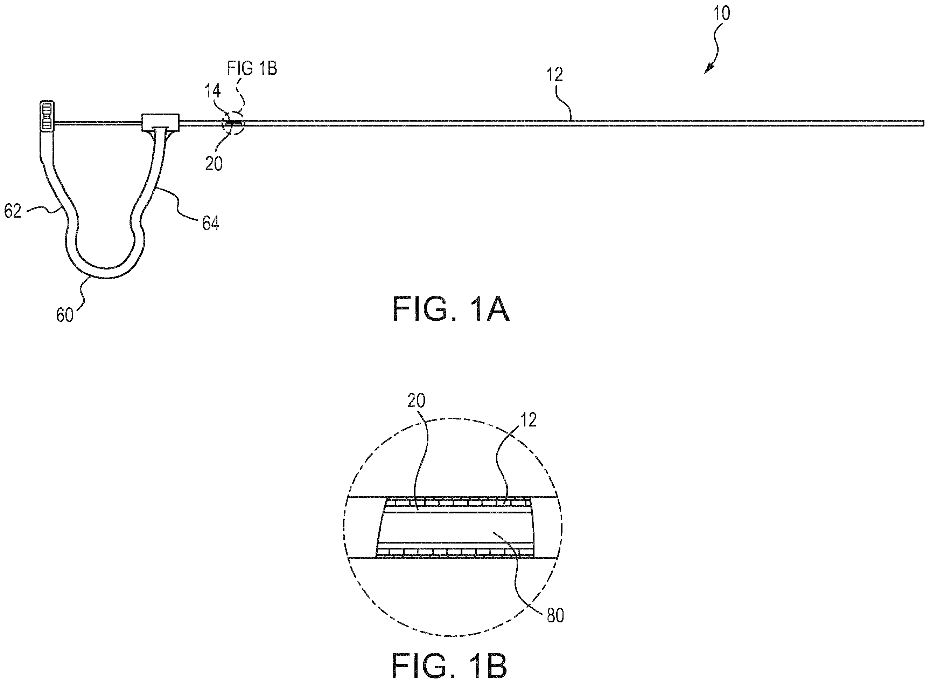

Referring to the figures, an example extractor 10, e.g., a percutaneous extractor, is useful in grasping and removing kidney stones from a patient. In example embodiments, the kidney stones are reduced in size, e.g., fragmented, by a procedure in which laser energy, electro-hydraulic energy, or sound energy is applied to reduce the kidney stones in size for easier removal, as described in greater detail below. Referring further to FIGS. 1A-3, for example, extractor 10 includes an outer cannula 12 forming a lumen 14 extending between a proximal end 16 and an opposing distal end 18 of outer cannula 12. In example embodiments, outer cannula 12 is a rigid, hollow tube that does not deflect appreciably in use. Extractor 10 may be used with a nephroscope, in which the surgeon inserts extractor 10 and outer cannula 12 into an appropriate channel in the nephroscope. The nephroscope allows the surgeon to view the operating field as the surgeon maneuvers the nephroscope and extractor 10 to capture and remove foreign bodies within the patient, such as kidney stones. In example embodiments, outer cannula 12 is sufficiently rigid for the surgeon to deflect and maneuver the nephroscope by using outer cannula 12 of extractor 10. Outer cannula 12 is made from a medically suitable material, such as a stainless steel or a plastic material with a minimal coefficient of friction, e.g., reinforced plastic, polyimide, or polytetrafluoroethylene (PTFE).

In example embodiments, outer cannula 12 has a length of 25.00 inches (63.50 centimeters (cm)) to 50.00 inches (127.00 cm), and, more particularly, a length of 28.00 inches (71.12 cm) to 45.00 inches (114.30 cm), suitable to allow the surgeon to reach the multiple poles of the patient's kidney by percutaneous introduction, for example. In alternative embodiments, outer cannula 12 may have any suitable length less than 25.0 inches or greater than 50.00 inches. Outer cannula 12 has an outer diameter of 0.010 inch (0.03 cm) to 0.118 inch (0.3 cm), and, more particularly, an outer diameter of 0.014 inch (0.0356 cm) to 0.063 inch (0.1400 cm). In example embodiments, outer cannula 12 has a wall thickness of at least 0.010 inch, and more particularly 0.014 inch, and even more particularly 0.015 inch. As the wall thickness increases, outer cannula 12 becomes more rigid. This rigidity enables the surgeon to control the nephroscope and to maneuver the nephroscope into a desired position. The surgeon thus delivers outer cannula 12 to the desired location within the operating field. An inner diameter of outer cannula 12 may range from 0.080 inch to 0.175 inch (from 6 Fr. to 13.5 Fr.). It is understood that in example embodiments the thickness of the outer cannula wall is maintained at a minimum of 0.015 inch, but an outer cannula wall thickness less than 0.015 inch may also be suitable.

In example embodiments, a sheath or other suitable coating or covering is positioned about an outer surface of outer cannula 12. In example embodiments, the sheath has a hydrophilic outer surface to facilitate movement of outer cannula 12 within the patient. For example, the sheath may be made of a suitable lubricious polymer material including, without limitation, a fluoropolymer liner, e.g., as polytetrafluoroethylene (PTFE) or Teflon® material, or another lubricious material such as polyethylene, polypropylene, nylon or polyurethane, for smooth movement of outer cannula 12 during introduction of extractor 10 into the patient and removal of extractor 10 from the patient. The sheath also protects the internal components of extractor 10, as described below. The sheath is desirably thin, for example, having a thickness of 0.10 millimeters (mm) to 0.4 mm (i.e., a thickness of 0.004 inch to 0.015 inch).

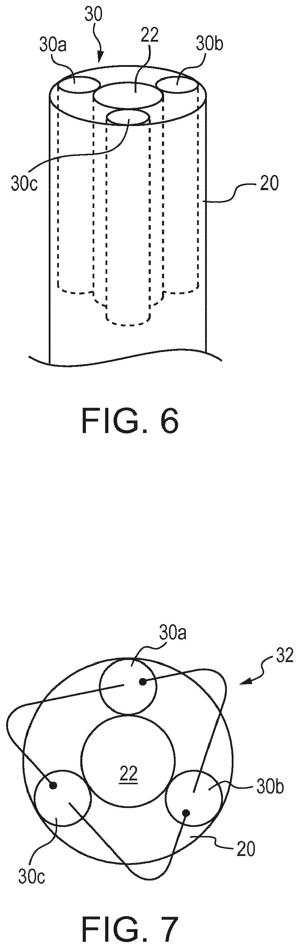

In example embodiments, an inner cannula 20 is slidably positioned within lumen 14 of outer cannula 12. An outer diameter of inner cannula 20 is sized such that inner cannula 20 is easily movable within lumen 14 of outer cannula 12 defining an inner diameter of outer cannula 12. In example embodiments, inner cannula 20 has a diameter of at least 0.065 inch (about 5 Fr); however, inner cannula 20 may have any suitable diameter provided that inner cannula 20 is movable within lumen 14 and capable of operating as described herein. Referring further to FIGS. 6 and 7, inner cannula 20 forms a central passage 22 along a longitudinal axis 24 of inner cannula 20 extending between a proximal end 26 and an opposing distal end 28 of inner cannula 20. In example embodiments, inner cannula 20 forms central passage 22 having a diameter of 0.05 inch to 0.070 inch, and, more particularly, a diameter of 0.061 inch to 0.065 inch (4.65 Fr. to 4.95 Fr.) with the inner cannula 20 having an outer diameter of 0.075 inch to 0.09 inch and, more particularly, an outer diameter of 0.082 inch to 0.084 inch. Inner cannula 20 also includes one or more, e.g., a plurality of, secondary passages 30 such as secondary passages 30a, 30b, and 30c, as shown in FIGS. 6 and 7. Secondary passages 30 extend along at least a portion of a length of inner cannula 20 between proximal end 26 and distal end 28.

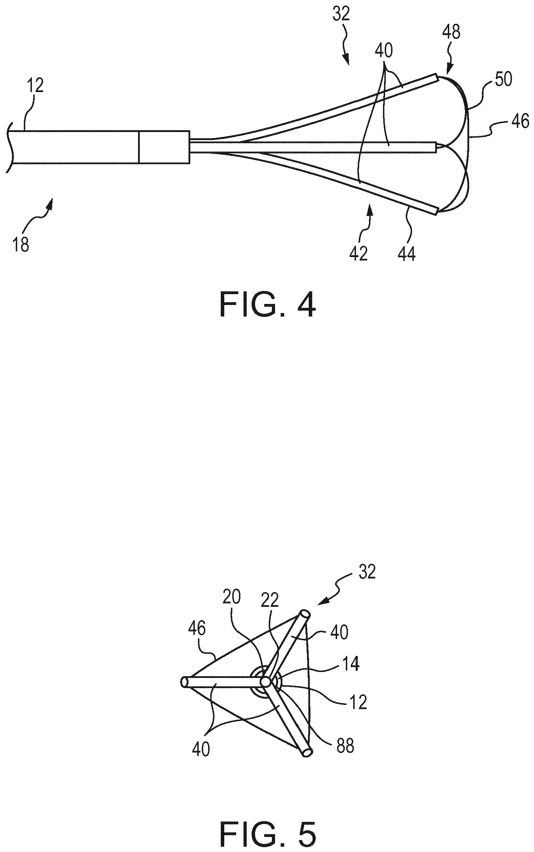

In example embodiments, an annularly expanding and retracting extraction mechanism 32 is operatively coupled to distal end 28 of inner cannula 20. Extraction mechanism 32 is operable to capture and extract kidney stones or other foreign bodies from within a cavity or a lumen of the patient. As shown in FIGS. 4 and 5, for example, a plurality of coextensive flexible tubular members, e.g., three flexible tubular members, generally indicated at 40 are disposed in an annular array at distal end 28. Flexible tubular members 40 are formed of a suitable plastic material, such as polyethylene, polypropylene, polyester, polyvinyl chloride, polyimide or another suitable plastic material. Each flexible tubular member 40 is positioned within an associated secondary passage 30 and extends along at least a portion of a length of inner cannula 20.

At distal end 28 of inner cannula 20, flexible tubular members 40 extend distally from corresponding secondary passage 30. An outwardly extending distal end section 42 of each flexible tubular member 40 constitutes a longitudinally fixed flexure element 44 forming a part of extraction mechanism 32. A plurality of wires 46, e.g., three wires, are associated with respective flexible tubular members 40. Wires 46 are preferably made of stainless steel, although other materials, e.g., electrically conducting or electrically insulating materials, may be used. Each wire 46 has a length of at least twice a length of flexible tubular members 40. Each wire 46 is bent at a midportion thereof so as to define a fixed wire section 48 and a movable wire section 50.

Each fixed wire sections 48 extends within a respective flexible tubular member 40 with the bend being disposed in a flexure position on the longitudinally fixed flexure elements 44 at a distal free end of flexible tubular member 40. Fixed wire sections 48 are fixed with respect to the respective flexible tubular member 40. Fixed wire section 48 transitions into an associated movable wire section 50 which extends distally from respective tubular member 40 and into an adjacent flexible tubular member 40. Movable wire section 50 extends through the adjacent flexible tubular member 40 and passes through the respective secondary passage 30 to operatively couple to a handle 60 of extractor 10 at proximal end 14 of outer cannula 12 and proximal end 26 of inner cannula 20. Handle 60 is configured for operating annularly expanding and retracting extraction mechanism 32.

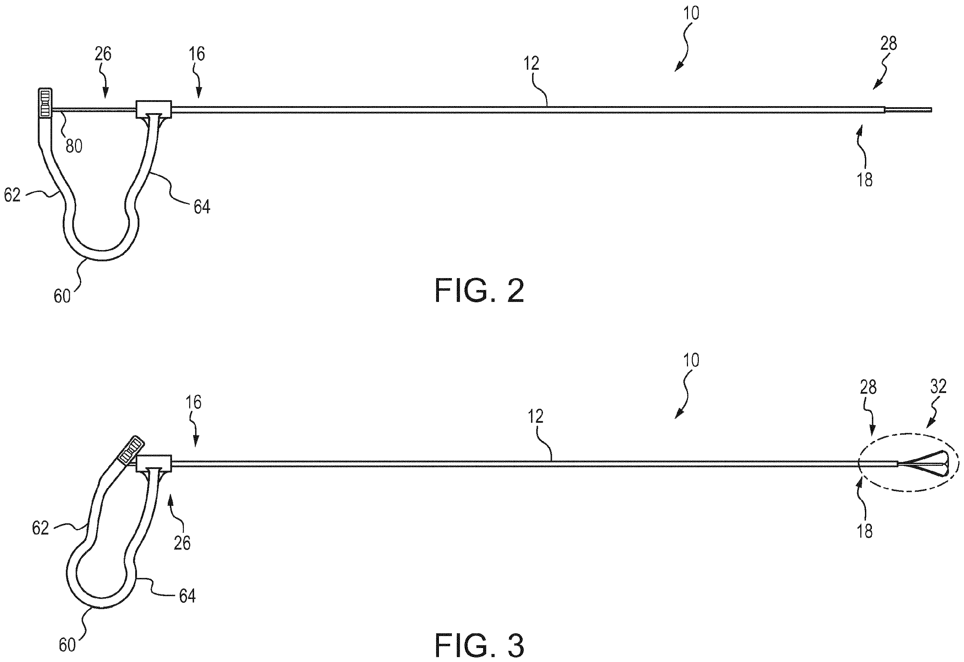

Referring further to FIGS. 1A-3, in example embodiments, handle 60 includes a first section 62 coupled to inner cannula 20 and a second section 64 coupled to outer cannula 12. Extraction mechanism 32 is operated by applying pressure to handle 60, e.g., squeezing handle 60, to urge first section 62 toward second section 64 to cause inner cannula 20 to translate through outer cannula 12 in a distal direction from a first position to a second position to extend extraction mechanism 32 distally from inner cannula 20, as shown, for example, in FIG. 2. Handle 60 is shown in FIGS. 1A and 2 in a "relaxed" configuration and handle 60 is shown in FIG. 3 in a "squeezed" configuration. Extraction mechanism 32 is extended from inner cannula 20 as shown when the operator or surgeon applies pressure and squeezes handle 60. When no pressure is applied to handle 60, handle 60 is in the relaxed configuration, and extraction mechanism 32 may be collapsed within inner cannula 20 (as shown in FIGS. 1A and 1B). Handle 60 is not meant for insertion into the body of a patient, but remains outside the body during procedures for removing foreign bodies, e.g., kidney stones, from the body of the patient. In example embodiments, handle 60 is made of a nylon material or another suitable plastic or composite material.

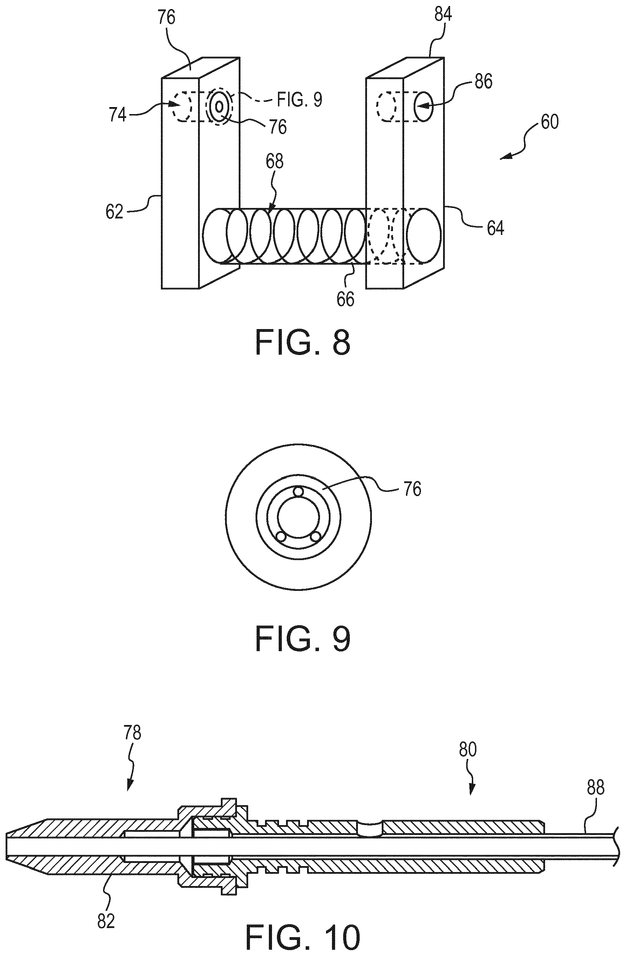

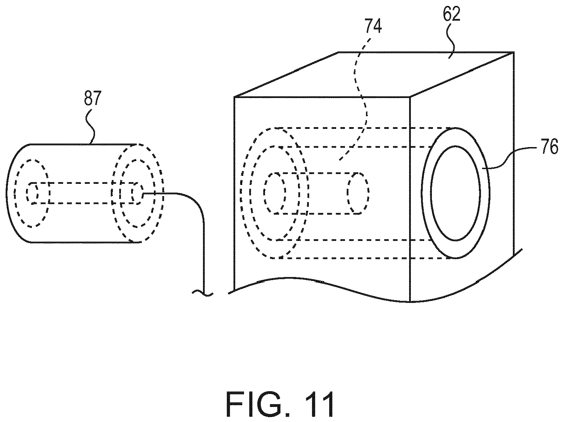

In an alternative example embodiment as shown in FIG. 8, handle 60 includes an intermediate section 66 between first section 62 and second section 64. Intermediate section 66 includes a biasing member 68, such as a spring or another suitable biasing member, operatively coupled to first section 62 and second section 64 to allow movement of first section 62 with respect to second section 64. Referring further to FIG. 8, first section 62 has an upper portion 72 with a first aperture 74 extending through a width of upper portion 72. A ring 76, such as shown in FIGS. 8 and 9, positioned within upper portion 72 about first aperture 74 is configured to receive each wire 46, e.g., each movable wire section 50, that extends through a respective secondary passage 30 in certain example embodiments. Ring 76 is also configured to receive, in certain example embodiments, a proximal end 78 of a laser assembly 80 to retain laser assembly 80 coupled to first section 62. As shown in FIG. 10, proximal end 78 of laser assembly 80 has a connector 82 configured to extend into first aperture 74 and securely couple to first section 62. Second section 64 of handle 60 includes an upper portion 84 having a second aperture 86 coaxially aligned with first aperture 74 of upper portion 72 of first section 62. Laser assembly 80 is coupled to upper portion 72 of first section 62 and extends through second aperture 86 in upper portion 84 of second section 64 to enter inner cannula 20. As first section 62 and second section 64 move towards each other, first aperture 74 and second aperture 86 remain coaxially aligned to facilitate movement of laser assembly 80 through inner cannula 20 in a distal direction, as well as an opposite proximal direction. In alternative embodiments, other suitable ablation and/or fragmenting devices, such as suitable ultrasonic or impact devices, e.g., percussive or concussive lithotripsy devices designed for transcatheter use, may be used instead of or in addition to laser assembly 80. As shown in FIG. 11, in certain example embodiments, a plug 87 couples ring 76 and/or inner cannula 20 to first section 62.

During such motion, extraction mechanism 32 and laser assembly 80 including a laser fiber 88 (shown in FIGS. 5 and 10), such as a holmium laser fiber, extend distally from distal end 28 of inner cannula 20 and distal end 18 of outer cannula 12. As extraction mechanism 32 expands to capture a kidney stone, laser assembly 80 moves in the distal direction to position laser assembly 80 in a proximate position to the kidney stone such that laser fiber 88 is operable to fragment the kidney stone into smaller pieces to facilitate removal of the kidney stone pieces with extraction mechanism 32. Because biasing member 68 biases first section 62 to move in an opposite proximal direction, once the force to move inner cannula 20 in the distal direction is removed, inner cannula 20 including extraction mechanism 32 is biased or urged to move in the proximal direction causing extraction mechanism 32 to collapse and at least partially retract into outer cannula 12. In example embodiments, laser assembly 80 extends through central passage 22. Laser assembly 80 includes a flexible laser fiber 88, such as a holmium laser fiber, configured to deliver focused holmium energy. In alternative embodiments, other suitable laser fibers may be used with laser assembly 80 or other suitable ablation or fragmenting devices, such as described above, may be used instead of laser assembly 80. In a particular embodiment, laser fiber 88 is a 940 micrometer (4.20 French) holmium laser fiber; however, laser fibers having different suitable sizes may be used.

In the use of extractor 10 in a percutaneous kidney stone removal procedure, the standard preparatory procedures and standard auxiliary equipment are used. Initially, a suitable percutaneous tract to the kidney in the patient's body is provided and an adequate visualization of the collecting system of the kidney by means of a scope is established through the percutaneous track. Outer cannula 12 is advanced through the percutaneous track to the target site. Next, distal end 28 of inner cannula 20 is advanced through outer cannula 12. Inner cannula 20 is advanced until distal end 28 of inner cannula 20 reaches the targeted area where the stone is to be removed. This advancement is accomplished by manually feeding extractor 10 through a working channel in the scope. When distal end 28 of inner cannula 20 reaches the target area as determined by visual inspection of the scope, the operator grasps handle 60 and moves first section 62 toward second section 64. An extent of the distal movement is sufficient to expand extraction mechanism 32, such as shown in FIGS. 3-5, to engage and capture the stone to be extracted.

As handle 60 is moved, the proximal ends of movable wire sections 50 are moved therewith. Because movable wire sections 50 are captured peripherally throughout their extent, the movement of the proximal ends causes the opposite distal ends to move outwardly of the distal ends of flexible tubular members 40 or fixed flexure elements 44. Because the distal ends of movable wire sections 50 are fixed to adjacent fixed flexure elements 44 by virtue of the fixture of integral fixed wire section 48 therewith, the movement of the distal end portions of movable wire sections 50 outwardly of fixed flexure elements 44 causes fixed flexure elements 44 to be flexed radially outwardly at the free ends and the outwardly extending end portions of movable wire sections 50 to flex arcuately outwardly beyond the distal free ends of fixed flexure elements 44.

When handle 60 reaches the deployed position, as shown in FIG. 3, the outwardly extending distal end portions of movable wire sections 50 are in arcuate configurations outwardly of the flexure positions of fixed flexure elements 44, as shown in FIGS. 4 and 5, for example. It can be seen that the flexure position of each fixed flexure element 44 is biased outwardly by two associated movable flexure elements, one of which is integrally connected with fixed wire section 48 therein and one of which is integral with movable wire section 50 therein. Because the two movable flexure elements associated with each fixed flexure element 44 have their opposite ends associated with the two adjacent fixed flexure elements 44, the flexural movement is imposed symmetrically upon each fixed flexure element 44 by the associated movable flexure elements. The result is that extraction mechanism 32 expands annularly from its retracted insertion position, as shown in FIG. 2, both radially outwardly and longitudinally outwardly. In its maximum expanded deployed position, as shown in FIG. 3, extraction mechanism 32 is defined at its outer portion by three longitudinally outwardly arcuately flexed movable flexure elements extending in an open annular series or array. In the embodiment shown, the movable flexure elements are in the form of wire sections constituting distal sections of movable wire sections 50, the remaining sections of which form continuing sections of wire 46. The inner portion of the maximally expanded deployed extraction mechanism 32 is defined by three radially outwardly flexed fixed flexure elements 44 extending from a position of confinement determined by the position of distal end 28 of inner cannula 20.

The deployment configuration whether maximal or less enables the operator to move the expanded extraction mechanism 32 longitudinally over the targeted kidney stone until it is captured therein. This longitudinal forward movement is a more natural movement to effect capture of the stone in the kidney's collecting system as compared with a lateral movement. Nevertheless, alternatively, it is possible to loop the most convenient arcuate flexure element over the stone to position it inside the deployed extraction mechanism 32. During the looping movement, it is noted that fixed flexure elements 44 which are not associated with the movable flexure element used to loop the stone as well as the movable flexure elements associated therewith provide structure to engage the stone as the looping movement progresses, thus establishing the full entry of the stone within extraction mechanism 32. The deployment movement is determined to take place in a coordinated relation with the position of the stone within the kidney collecting system. The advancing longitudinal movement or the looping movement can be a coordinated part of the deployment movement or fully sequential. In this way, either the annular series of outwardly arcuately flexed movable flexure elements are moved around the stone or the selected movable flexure element more or less is reached out and looped over the stone. In this coordinated movement, it is noted that there are no sharp points ever presented to deal with which might start hemorrhaging. Other suitable extraction mechanisms, e.g., other baskets, can be used to replace extraction mechanism 32 as described herein.

Once the stone is positioned within the expanded extraction mechanism 32, the operator moves handle 60 in a proximal rearward direction away from the deployed position toward the insertion position shown in FIGS. 1 and 2. This rearward movement of handle 60 effectively retracts the movable flexure elements back into fixed flexure elements 44 of extraction mechanism 32. As this movement progresses, the arcuate extent of the movable flexure elements becomes smaller and the flexure positions at the free ends of fixed flexure elements 44 move radially inwardly. This progressive movement has the effect of engaging the stone within fixed flexure elements 44. As the movable flexure elements continue to move within fixed flexure elements 44, the outer portion of extraction mechanism 32 is retracted both radially and longitudinally inwardly. The retracting outer portion of extraction mechanism 32 including the movable flexure elements and free ends of fixed flexure elements 44 alternately move into tight gripping engagement with the outer portion of the stone. This tight gripping engagement biases the stone inwardly into a tighter captured relationship within fixed flexure elements 20.

With the stone tightly engaged within extraction mechanism 32, laser assembly 80 is utilized to fragment the stone to facilitate removing smaller pieces of stone from within the patient's kidney. Once the stone has been fragmented, inner cannula 20 can be withdrawn from the patient outwardly of the installed percutaneous tract. Note that during this fixed withdrawing movement the broader fixed flexure elements 44 are leading. The use of laser assembly 80 in cooperation with extraction mechanism 32 allows for localizing the area in which the kidney stone resides, as well as producing smaller stone fragments that are easier to remove. This also minimizes surgical time and reduces the need for clear visualization during the procedure. Extraction mechanism 32 and a placement of laser assembly 80 in central passage 22allows the surgeon to hold the stone directly in line with laser fiber 88 with the wires of extraction mechanism outside the line of the laser fiber sight, reducing the incidence of damage to extraction mechanism 32.

Although the subject matter has been described in language specific to structural features and/or methodological acts, it is to be understood that the subject matter defined in the appended claims is not necessarily limited to the specific features or acts described. Rather, the specific features and acts are disclosed as illustrative forms of implementing the claims.

One skilled in the art will realize that a virtually unlimited number of variations to the above descriptions are possible, and that the examples and the accompanying figures are merely to illustrate one or more examples of implementations. For example, in an embodiment there is provided an extractor, comprising: an outer cannula having a lumen extending between a proximal end and an opposite distal end of the outer cannula; an inner cannula movably positioned within the outer cannula, the inner cannula having a proximal end configured to be retained exteriorly of a patient and a distal end configured to be inserted into the patient, the inner cannula including a central passage along a longitudinal axis of the inner cannula extending between a proximal end and an opposing distal end of the inner cannula and a plurality of secondary passages positioned about the central passage and extending along at least a portion of a length of the inner cannula between the proximal end and the distal end of the inner cannula; and, an annularly expanding extraction mechanism at the distal end of the inner cannula, the extraction mechanism operable to capture and extract kidney stones from within a lumen of a patient. The plurality of secondary passages may extend from the proximal end of the inner cannula to the distal end of the inner cannula. The inner cannula may be movable within the outer cannula by sliding. The annularly expanding extraction mechanism may comprise a plurality of fixed flexure elements positioned about a periphery of the central passage in an annular array at a confining fixed position and having a flexure position spaced longitudinally outwardly therefrom. It may also comprise a plurality of corresponding movable flexure elements, each movable flexure element of the plurality of corresponding movable flexure elements may have an end fixed with respect to the flexure position of a corresponding fixed flexure element of the plurality of fixed flexure elements. The movable flexure elements may extend from this fixed end in a longitudinally movable and transversely confined relation to a receiving portion of an adjacent fixed flexure element of the plurality of fixed flexure elements. Each movable flexure element may extend through the adjacent fixed flexure element and a respective secondary passage of the plurality of secondary passages to couple to the first section of the handle. In an embodiment there is provided an extractor which includes a rigid outer cannula forming a lumen extending between a proximal end and an opposing distal end of the outer cannula. An inner cannula is slidably positioned within the lumen of the outer cannula. The inner cannula forms a central passage along a longitudinal axis of the inner cannula extending between a proximal end and an opposing distal end of the inner cannula. A plurality of secondary passages are positioned about the central passage and extend along at least a portion of a length of the inner cannula between the proximal end and the distal end of the inner cannula. An annularly expanding and retracting extraction mechanism at the distal end of the inner cannula is operable to capture and extract kidney stones from within a lumen of a patient. The extractors described in this paragraph may comprise the features of any one or more of claims 2 to 15, and/or any of the other extractors described herein.

It will be understood by those skilled in the art that various other modifications can be made, and equivalents can be substituted, without departing from claimed subject matter. Additionally, many modifications can be made to adapt a particular situation to the teachings of claimed subject matter without departing from the central concept described herein. Therefore, it is intended that claimed subject matter not be limited to the particular embodiments disclosed, but that such claimed subject matter can also include all embodiments falling within the scope of the appended claims, and equivalents thereof.

In the detailed description above, numerous specific details are set forth to provide a thorough understanding of claimed subject matter. However, it will be understood by those skilled in the art that claimed subject matter can be practiced without these specific details. In other instances, methods, devices, or systems that would be known by one of ordinary skill have not been described in detail so as not to obscure claimed subject matter.

Reference throughout this specification to "one embodiment" or "an embodiment" can mean that a particular feature, structure, or characteristic described in connection with a particular embodiment can be included in at least one embodiment of claimed subject matter. Thus, appearances of the phrase "in one embodiment" or "an embodiment" in various places throughout this specification are not necessarily intended to refer to the same embodiment or to any one particular embodiment described. Furthermore, it is to be understood that particular features, structures, or characteristics described can be combined in various ways in one or more embodiments. In general, of course, these and other issues can vary with the particular context of usage. Therefore, the particular context of the description or the usage of these terms can provide helpful guidance regarding inferences to be drawn for that context.

## Claims

1. An extractor, comprising:
an outer cannula having a lumen extending between a proximal end and an opposite distal end of the outer cannula;
an inner cannula movably positioned within the outer cannula, the inner cannula having a proximal end configured to be retained exteriorly of a patient and a distal end configured to be inserted into the patient, the inner cannula including:
a central passage extending along a longitudinal axis of the inner cannula between the proximal end and the distal end; and
a plurality of secondary passages positioned about the central passage, each secondary passage of the plurality of secondary passages extending between the proximal end and the distal end of the inner cannula;
a handle having a first section coupled to the proximal end of the inner cannula and a second section coupled to the proximal end of the outer cannula, the handle movable between a first position and a second position to move the inner cannula within the outer cannula; and
an annularly expanding extraction mechanism operatively coupled to the distal end of the inner cannula, the annularly expanding extraction mechanism comprising:
a plurality of fixed flexure elements positioned about a periphery of the central passage in an annular array at a confining fixed position and having a flexure position spaced longitudinally outwardly therefrom; and
a plurality of corresponding movable flexure elements, each movable flexure element of the plurality of corresponding movable flexure elements having an end fixed with respect to the flexure position of a corresponding fixed flexure element of the plurality of fixed flexure elements and extending therefrom in a longitudinally movable and generally transversely confined relation to a receiving portion of an adjacent fixed flexure element of the plurality of fixed flexure elements, wherein each movable flexure element extends through the adjacent fixed flexure element and a respective secondary passage of the plurality of secondary passages to couple to the first section of the handle.

2. The extractor of claim 1, further comprising a laser assembly movably positioned within the central passage, the laser assembly having a laser fiber configured to extend distally with respect to the distal end of the inner cannula as the handle is moved to the second position.

3. The extractor of claim 1 or 2, wherein the handle further comprises a first aperture in the first section and a ring positioned about the first aperture, and each movable flexure element is coupled to the ring, for example further comprising a laser assembly movably positioned within the central passage, wherein the ring forms a passage coaxially aligned with the central passage, the passage configured to receive a proximal end portion of the laser assembly.

4. The extractor of any preceding claim, wherein the handle is operatively coupled to the extraction mechanism such that a manual movement of the first section towards the second section in a first direction will effect a movement of the plurality of movable flexure elements in an outward direction with respect to the corresponding fixed flexure element to extend in an arcuately flexed condition beyond the flexure positions of the plurality of fixed flexure elements to cause the plurality of fixed flexure elements to flex transversely outwardly and create an expanded condition defined by transversely outwardly flexed fixed flexure elements interconnected by an annular series of arcuately movable flexure elements for example, wherein the handle is configured so that a manual movement of the handle in an opposite second direction will effect a movement of the movable flexure elements when in the expanded condition in a direction inwardly with respect to the corresponding fixed flexure elements to cause the expanded condition to progressively retract during which the transversely outwardly flexed fixed flexure elements are progressively less flexed transversely outwardly and the arcuately flexed movable flexure elements have a progressively less arcuate extent.

5. The extractor of any preceding claim, wherein as the first section is urged toward the second section, the inner cannula translates through the lumen of the outer cannula in a distal direction to extend the extraction mechanism distally from the inner cannula, for example wherein the handle further comprises an intermediate section between the first section and the second section, the intermediate section including a biasing member operatively coupled to the first section and the second section to allow movement of the first section with respect to the second section.

6. The extractor of claim 5, wherein the first section has an upper portion with a first aperture extending through a width of the upper portion and a ring positioned within the upper portion about the first aperture, wherein the ring is configured to receive each movable wire section that extends through a respective secondary passage.

7. The extractor of claim 6, further comprising a laser assembly, wherein the ring is configured to receive a proximal end of the laser assembly to retain the laser assembly coupled to the first section, the proximal end of the laser assembly having a connector configured to extend into the first aperture and securely couple to the first section.

8. The extractor of claim 7, wherein the second section of the handle includes an upper portion having a second aperture coaxially aligned with first aperture of the upper portion of the first section, wherein the laser assembly is coupled to the upper portion of the first section and extends through the second aperture in the upper portion of the second section to enter the inner cannula and as the first section and the second section move towards each other, the first aperture and the second aperture remain coaxially aligned to facilitate movement of the laser assembly through the inner cannula in a distal direction.

9. An extractor, comprising:
a rigid outer cannula forming a lumen extending between a proximal end and an opposing distal end of the outer cannula;
an inner cannula slidably positioned within the lumen of the outer cannula, the inner cannula forming a central passage along a longitudinal axis of the inner cannula extending between a proximal end and an opposing distal end of the inner cannula and a plurality of secondary passages positioned about the central passage and extending along at least a portion of a length of the inner cannula between the proximal end and the distal end of the inner cannula; and
an annularly expanding and retracting extraction mechanism at the distal end of the inner cannula, the extraction mechanism operable to capture and extract kidney stones from within a lumen of a patient.

10. The extractor of claim 9, wherein the annularly expanding and retracting extraction mechanism comprises a plurality of coextensive flexible tubular members disposed in an annular array at the distal end of the inner cannula, each flexible tubular member of the plurality of coextensive flexible tubular members is positioned within an associated secondary passage of the plurality of secondary passages, each flexible tubular member extends distally from the corresponding secondary passage and each wire of a plurality of wires is associated with a respective flexible tubular member of the plurality of coextensive flexible tubular members, each wire defines a fixed wire section and a movable wire section, each fixed wire section extends within a respective flexible tubular member and each movable wire section extends distally from the respective flexible tubular member and into an adjacent flexible tubular member.

11. The extractor of claim 10, wherein each movable wire section extends through the adjacent flexible tubular member and passes through a respective secondary passage to operatively couple to the handle.

12. The extractor of claim 9, 10, or 11, further comprising a laser assembly extending through the central passage, the laser assembly includes a flexible laser fiber configured to deliver focused holmium energy at the distal end of the inner cannula.

13. The extractor of any of claims 9 to 12, further comprising a handle configured to operate the extraction mechanism, the handle includes a first section coupled to the inner cannula and a second section coupled to the outer cannula, wherein as the first section is urged toward the second section, the inner cannula translates through the outer cannula in a distal direction to extend the extraction mechanism distally from the inner cannula, for example wherein the handle further comprises an intermediate section between the first section and the second section, the intermediate section includes a biasing member operatively coupled to the first section and the second section to allow movement of the first section with respect to the second section.

14. The extractor of claim 13, wherein the first section has an upper portion with a first aperture extending through a width of the upper portion and a ring positioned within the upper portion about the first aperture, wherein the ring is configured to receive each movable wire section that extends through a respective secondary passage, for example wherein the ring is configured to receive a proximal end of a laser assembly to retain the laser assembly coupled to the first section, the proximal end of the laser assembly having a connector configured to extend into the first aperture and securely couple to the first section.

15. The extractor of claim 13 or 14, wherein the second section of the handle includes an upper portion having a second aperture coaxially aligned with first aperture of the upper portion of the first second, wherein the laser assembly is coupled to the upper portion of the first section and extends through the second aperture in the upper portion of the second section to enter the inner cannula and as the first section and the second section move towards each other, the first aperture and the second aperture remain coaxially aligned to facilitate movement of the laser assembly through the inner cannula in a distal direction.
